# EUROPÄISCHE PATENTSCHRIFT

(11) **EP 3 970 756 B1**
(45) Veröffentlichungstag und Bekanntmachung des Hinweises auf die Patenterteilung: **06.09.2023**
(21) Anmeldenummer: 20197325.2
(22) Anmeldetag: 22.09.2020
(51) Int. Cl.: A61L 2/10, G05D 1/02, B61D 49/00, B61D 33/00, B08B 7/00, A61L 2/24

(54) **VERFAHREN ZUM DESINFIZIEREN VON VERKEHRSMITTELN UND VORRICHTUNGEN ZUR DURCHFÜHRUNG DESSELBEN**
METHOD FOR DISINFECTING TRANSPORT MEANS AND DEVICES FOR CARRYING OUT THE METHOD
PROCÉDÉ DE DÉSINFECTION DES MOYENS DE TRANSPORT ET DISPOSITIFS DE MISE EN OEUVRE DUDIT PROCÉDÉ

(43) Veröffentlichungstag der Anmeldung: 23.03.2022
(73) Patentinhaber: Siemens Mobility GmbH, 81739 München (DE)
(72) Erfinder: Rahn, Karsten, 38162 Cremlingen (DE)
(74) Vertreter: Siemens Patent Attorneys

(56) Entgegenhaltungen:
- WO-A1-2016/164362
- WO-A1-2019/068189
- DE-A1-102012 006 972
- US-A1- 2019 030 195
- US-A1- 2020 206 375

## Beschreibung

Die Erfindung betrifft ein Verfahren zum Desinfizieren eines Benutzerraumes in einem Fahrzeug, bei dem in dem Benutzerraum ein Desinfektionsvorgang durchgeführt wird. Außerdem betrifft die Erfindung ein Fahrzeug, das eingerichtet ist, ein Verfahren zum Desinfizieren eines Benutzerraumes des Fahrzeugs durchzuführen, indem in dem Benutzerraum ein Desinfektionsvorgang durchgeführt wird. Weiterhin betrifft die Erfindung eine mobile Einheit, die eingerichtet ist, ein Verfahren zum Desinfizieren eines Benutzerraumes des Fahrzeugs durchzuführen, indem in dem Benutzerraum ein Desinfektionsvorgang durchgeführt wird. Zuletzt betrifft die Erfindung ein Computerprogrammprodukt sowie eine Bereitstellungsvorrichtung für dieses Computerprogrammprodukt, wobei das Computerprogrammprodukt mit Programmbefehlen zur Durchführung dieses Verfahrens ausgestattet ist.

Dokument WO 2019/068189 A1 beschreibt eine Beleuchtungsanordnung für ein Flugzeug und umfasst eine Quelle sichtbaren Lichts, die sichtbares Licht erzeugt, und eine Quelle ultravioletten Lichts, die ultraviolettes Licht erzeugt. Die Quelle für sichtbares Licht ist neben der Quelle für ultraviolettes Licht angeordnet. Die sichtbare Lichtquelle beleuchtet einen ersten Beleuchtungsbereich mit dem sichtbaren Licht, wenn die Beleuchtungsbaugruppe in einem ersten Betriebsmodus arbeitet. Die Ultraviolettlichtquelle beleuchtet einen zweiten Beleuchtungsbereich mit dem Ultraviolettlicht, wenn die Beleuchtungsanordnung in einem zweiten Betriebsmodus arbeitet. Der erste Beleuchtungsbereich überlappt im Wesentlichen den zweiten Beleuchtungsbereich.

Dokument DE 10 2012 006972 A1 betrifft eine Reinigungseinrichtung zum automatischen oder halbautomatischen Reinigen von Fahrzeuginnenräumen, zum Beispiel eines Schienenfahrzeuges, und weist wenigstens eine Strahlungsvorrichtung, welche Strahlung in wenigstens einem vorbestimmten Wellenlängenbereich zwischen etwa 150 nm und etwa 350 nm emittiert, und eine Befestigungseinrichtung zum Montieren der wenigstens einen Strahlungsvorrichtung in einem Fahrzeuginnenraum auf. Die von der wenigstens einen Strahlungsvorrichtung emittierte Strahlung ist im Wesentlichen auf wenigstens einen Einrichtungsgegenstand des Fahrzeuginnenraums und/oder in den Fahrzeuginnenraum gerichtet.

In Dokument US 2019/030195 A1 wird ein Kabinendesinfektionssystem für ein Flugzeug beschrieben. Ein Erfassungsmittel zum Erfassen der Anwesenheit von Personal in einer oder mehreren Zonen, ein prädiktives Erfassungsmittel zum Erfassen der Bewegungsrichtung einer Person und zum Vorhersagen, ob diese Person eine zu desinfizierende Zone während einer geplanten Desinfektion belegen wird, und eine Steuerung zum Steuern des Betriebs der UVC-Strahlungsquellen in Abhängigkeit von der Erfassung von Personal in dieser Zone und den prädiktiven Erfassungsmitteln.

Dokument US 2020/206375 A1 betrifft ein tragbares UV-C-Desinfektionsgerät, Verfahren und System für keimtötende Ultraviolettbestrahlung. UV-C-Sensoren sind konfiguriert, um die Menge an UV-C-Licht oder nahem UV-C-Licht von einer Zieloberfläche zu messen. Eine Steuerung kann kommunikativ mit den UV-C-Sensoren verbunden sein, um die Menge an UV-C-Strahlung zu bestimmen, die von den UV-C-Sensoren gesammelt wird. Die Steuerung enthält in einem Speicher gespeicherte Anweisungen entsprechend der gesammelten UV-C-Strahlungsmenge, die einer Abtötungsdosis für die Oberflächendesinfektion entspricht.

Dokument WO 2016/164362 A1 beschreibt das Desinfizieren von Oberflächen an einem Ort im Zusammenhang mit Flugzeugen. Es gibt mehrere Sitzreihen. Eine Desinfektionsvorrichtung umfasst einen beweglichen Körper, der so konfiguriert ist, dass er sich entlang des Gangs des Flugzeugs bewegt. Ein Arm erstreckt sich von der Desinfektionsvorrichtung seitlich von dem beweglichen Körper über die Sitze, und eine an der Desinfektionsvorrichtung angebrachte UV-Strahlungsquelle wird über die Sitze gerichtet, wodurch Oberflächen im Fahrgastbereich UV-Strahlung ausgesetzt werden. Es gibt einen Datensammler, um Informationen im Flugzeug zu sammeln.

Die Aufgabe der Erfindung besteht darin, ein Verfahren sowie Vorrichtungen zum Desinfizieren eines Benutzerraumes mit einem Roboter in einem Fahrzeug anzugeben, mit dem bzw. mit denen sich eine Desinfektion des Fahrzeugs durchführen lässt ohne Benutzer des Fahrzeugs (Passagiere sowie bedienen Personal) durch die Desinfektion und Hindernisse, die dem Roboter den Weg versperren, zu gefährden. Außerdem besteht die Aufgabe der Erfindung darin, ein Computerprogrammprodukt sowie eine Bereitstellungsvorrichtung für dieses Computerprogrammprodukt anzugeben, mit dem das vorgenannte Verfahren durchgeführt werden kann.

Diese Aufgabe wird mit dem eingangs angegebenen Anspruchsgegenstand (Verfahren zum Desinfizieren) erfindungsgemäß dadurch gelöst, dass der Desinfektionsvorgang automatisch durchgeführt wird und vor dem automatischen Durchführen der Desinfektionsmaßnahmen rechnergestützt (evtl. auch rechnergestützt mit Einbindung oder Unterstützung von Personal) eine Beurteilung durchgeführt wird, ob der Benutzerraum frei von Benutzern ist, wobei die Desinfektionsvorgang nur gestartet wird, wenn die Beurteilung zu dem Ergebnis geführt hat, dass der Benutzerraum frei von Benutzern ist.

Erfindungsgemäß wird der Desinfektionsvorgang nur dann gestartet, wenn Gefährdungen für Personen im Benutzerraum ausgeschlossen werden können. Hierzu ist die gemäß der Erfindung vorgeschlagene Maßnahme, dass eine Beurteilung durchgeführt wird, ob der Benutzerraum frei von Benutzern ist, von vorrangiger Bedeutung. So kann sichergestellt werden, dass ein Gesundheitsrisiko, welches von dem Desinfektionsvorgang ausgeht, ausgeschlossen werden kann. Ein Gesundheitsrisiko kann physikalischer oder chemischer Natur sein. Dies hängt davon ab, welche Desinfektionsmaßnahmen durchgeführt werden. Es können Chemikalien oder Strahlungen zum Einsatz kommen (hierzu im Folgenden noch mehr).

Unter einem Benutzerraum wird insbesondere der Fahrgastraum des öffentlichen Verkehrsmittels verstanden. Allerdings ist auch das Zugpersonal als Benutzer des Verkehrsmittels zu verstehen. Auch die dem Personal zur Verfügung stehenden Räume (Servicebereich, Führerstand) sollen im Sinne der Erfindung als Benutzerraum verstanden werden. Außerdem ist der Benutzerraum nicht auf den Innenraum des Fahrzeugs beschränkt. Hat das Fahrzeug beispielsweise Öffnungen oder Fenster, können schädliche Substanzen oder Strahlung daraus austreten und außerhalb des Fahrzeugs befindliche Benutzer schädigen. Der Benutzerraum kann somit auch einen Raum um das Fahrzeug herum umfassen.

Eine rechnergestützte Beurteilung bedeutet nicht zwangsläufig, dass diese ausschließlich rechnergestützt stattfinden muss. Es kann beispielsweise auch Sichtprüfung durch Personal durchgeführt werden und im Anschluss eine Betätigung eines sicheren Tasters oder ein anderes sicheres Kommando erfolgen. Hierbei ist allerdings eine Schnittstelle notwendig, damit der Vorgang der Beurteilung rechnergestützt ablaufen kann.

Die Desinfektion stellt einen wichtigen Teil der Infektionsbekämpfung insbesondere zu Zeiten der Ausbreitung einer Pandemie dar. Im Sinne der Erfindung bedeutet Desinfektion, totes oder lebendes Material in einen Zustand versetzen, dass es nicht mehr infizieren kann.

Zur Desinfektion können chemische oder physikalische Verfahren eingesetzt werden. Es gibt verschiedene Listen mit geprüften Desinfektionsmitteln und -verfahren, in denen diese nach verschiedenen Einsatzbereichen aufgeführt sind.

Desinfektionsmittel und -Verfahren sind somit auch für Flächen-, Wäsche- und Raumdesinfektion sowie Desinfektion von Abfällen bekannt. Diese Maßnahmen gehören zum Teil zur Basishygiene und sind daher dazu geeignet, auch das Infektionsrisiko in öffentlichen Verkehrsmitteln zu verringern.

Von Desinfektion spricht man bei einer Keimreduktion in einem festgelegten Testverfahren mit bestimmten Prüfkörpern um einen Faktor von mindestens 10⁻⁵, das heißt, dass von ursprünglich 1.000.000 vermehrungsfähigen Keimen (sogenannten koloniebildende Einheiten (KbE)) nicht mehr als zehn überleben. Im Rahmen dieser Erfindung soll von einer vollständigen Desinfektion gesprochen werden, wenn bei der Maßnahme der genannte Faktor von zehn hoch -5 erreicht wird. Allerdings wird die Ansteckungsgefahr in öffentlichen Verkehrsmitteln bereits auch dann reduziert, wenn der Wert einer vollständigen Desinfektion (gültig zum Beispiel für Krankenhäuser) in dem öffentlichen Verkehrsmittel nicht erreicht wird. In diesem Fall soll im Rahmen dieser Erfindung von einer Desinfektion gesprochen werden.

Die erfindungsgemäße obligatorische Überprüfung, ob der Benutzerraum frei von Benutzern ist, ermöglicht es vorteilhaft, das Verfahren mit der erforderlichen Sicherheit nur anzuwenden, wenn eine Gefährdung von Benutzern ausgeschlossen werden kann. Als Benutzer sollen im weiteren Sinne nicht nur Menschen, sondern auch größere Tiere verstanden werden, deren Anwesenheit beispielsweise durch Scanner festgestellt werden kann.

Eine andere Möglichkeit besteht darin, dass eine Kontrolle des Benutzerraums durch das Betreiberpersonal des Fahrzeugs oder Personal am Bahnsteig durchgeführt wird. Dies ist dann eine manuelle Kontrolle, die allerdings dahingehend rechnergestützt erfolgt, dass eine Eingabe erfolgt, sobald die Freiheit des Benutzerraums von Benutzern festgestellt wurde, zum Beispiel über einen Taster.

Die Freiheit des Benutzerraums von Benutzern wird ohne weiteres beispielsweise auch nach Betriebsschluss festgestellt. Wenn die Fahrzeuge dann beispielsweise in ein Depot einfahren, kann auf diese Weise auch durch die das Fahrzeug umgebenden Räumlichkeiten sichergestellt werden, dass Benutzer von dem Benutzerraum ferngehalten werden können.

Erfindungsgemäß ist vorgesehen, dass der Desinfektionsvorgang durchgeführt wird, indem durch den Benutzerraum eine mobile Einheit geführt wird.

Vorzugsweise kann die mobile Einheit eine Desinfektion durch Strahlung vornehmen. Dies ist besonders einfach möglich, weil die mobile Einheit dann beispielsweise durch den Mittelgang eines Personen-Schienenfahrzeugs geführt werden kann. Aber es ist auch möglich, zusätzlich weitere Desinfektionsmittel einzusetzen. Beispielsweise können die Haltestangen, die den Mittelgang flankieren, mit einer Desinfektionslösung behandelt werden.

Ein weiterer Vorteil der Verwendung einer mobilen Einheit ist, dass diese für mehrere Fahrzeuge verwendet werden kann und somit gut ausgelastet werden kann. Es ist damit auch möglich, Fahrzeuge zu desinfizieren, die bisher nicht mit einem fest installierten Desinfektionssystem ausgestattet sind.

Außerdem ist erfindungsgemäß vorgesehen, dass die mobile Einheit als Roboter ausgeführt ist, der durch ein in den Roboter integriertes Steuersystem geführt wird.

Vorteilhaft ist es damit möglich, die mobile Einheit selbst gesteuert durch das Schienenfahrzeug zu bewegen. Der Vorteil ist, dass der Roboter dann beispielsweise Hindernissen ausweichen kann, und sich so in Fahrzeugen mit unterschiedlichen topographischen Gegebenheiten bewegen kann.

Möglich ist es, dass der Roboter mit einer eigenen Steuereinrichtung versehen ist, die der ATC eines Schienenfahrzeugs nachempfunden ist. Auf diesem Wege ist es vorteilhaft möglich, dass der Roboter mit der ATC des Fahrzeugs, durch das er sich bewegt, mittels der im Bahnbetrieb üblichen Kommunikationswege und Kommunikationsstandards kommuniziert.

Wesentlich für die Erfindung ist es, dass der Roboter über eine Schnittstelle mit der ATC-Einrichtung des Fahrzeugs kommuniziert und auch gesteuert wird.

Gemäß dieser Ausgestaltung der Erfindung ist somit vorgesehen, dass die ATC-Einrichtung des Fahrzeugs auch die Steuerung des Roboters zumindest weitgehend übernimmt. Hierbei sind selbstverständlich verschiedene Varianten möglich.

Beispielsweise kann die globale Steuerung, die eine Kenntnis der topographischen Gegebenheiten der des Fahrzeugs voraussetzt, durch die ATC-Einrichtung des Fahrzeugs übernommen werden. Eine Kontrolle, dass unvorhergesehene Hindernisse im Weg stehen, könnte beispielsweise durch den Roboter mit einer bordeigenen Sensorik und Steuerung selbst durchgeführt werden, so dass dieser für den Fall, dass der Weg, der für ihn bestimmt ist, versperrt ist, selbstständig anhält.

Unter "rechnergestützt" oder "computerimplementiert" kann im Zusammenhang mit der Erfindung eine Implementierung des Verfahrens verstanden werden, bei dem mindestens ein Computer oder Prozessor mindestens einen Verfahrensschritt des Verfahrens ausführt.

Der Ausdruck "Rechner" oder "Computer" deckt alle elektronischen Geräte mit Datenverarbeitungseigenschaften ab. Computer können beispielsweise Personal Computer, Server, Handheld-Computer, Mobilfunkgeräte und andere Kommunikationsgeräte, die rechnergestützt Daten verarbeiten, Prozessoren und andere elektronische Geräte zur Datenverarbeitung sein, die vorzugsweise auch zu einem Netzwerk zusammengeschlossen sein können.

Unter einer "Speichereinheit" kann im Zusammenhang mit der Erfindung beispielsweise ein computerlesbarer Speicher in Form eines Arbeitsspeichers (engl. Random-Access Memory, RAM) oder Datenspeichers (Festplatte oder Datenträger) verstanden werden.

Als "Schnittstellen" können hardwaretechnisch, beispielsweise kabelgebunden oder als Funkverbindung, und/oder softwaretechnisch, beispielweise als Interaktion zwischen einzelnen Programmmodulen oder Programmteilen eines oder mehrerer Computerprogramme, realisiert sein.

Als "Programmmodule" sollen einzelne Funktionseinheiten verstanden werden, die die Abarbeitung des erfindungsgemäßen Programmablaufs in Verfahrensschritten ermöglichen. Diese Funktionseinheiten können in einem einzigen Computerprogramm oder in mehreren miteinander kommunizierenden Computerprogrammen verwirklicht sein. Die hierbei realisierten Schnittstellen können softwaretechnisch innerhalb eines einzigen Prozessors umgesetzt sein oder hardwaretechnisch, wenn mehrere Prozessoren zum Einsatz kommen.

Gemäß einer Ausgestaltung der Erfindung ist vorgesehen, dass die Aktivierung eines Desinfektionsvorgangs durch eine fahrzeugseitige automatische Zugsteuerung (im Folgenden kurz ATC oder ATC-Einrichtung) eines Schienenfahrzeugs mittels eines signaltechnisch sicheren Ausgangs vorgenommen wird, nachdem diese ein Signal erhalten hat, welches darauf hinweist, dass der Benutzerraum frei von Benutzern ist. Die ATC muss nicht allein durch den betreffenden Zug durchgeführt werden, sondern kann teilweise auch durch streckenseitige Einrichtungen unterstützt werden. Beispiele für ATC-Einrichtungen sind folgende Zugkontrollsysteme (TCS): European Train Control System (ETCS), Communications Based Train Control (CBTC), Positive Train Control (PTC) und Konventionelle Zugbeeinflussung oder Conventional Train Control (CTC).

Der Begriff ATC ist in dem Sinne zu verstehen, dass der Zug zumindest teilweise automatisch gesteuert wird, wobei die Aktivierung des Desinfektionsvorganges in den automatischen Anteil des Vorganges der Zugsteuerung integriert ist. Der Vorgang, der einer Aktivierung vorausgeht, um die Voraussetzungen für die Aktivierung zu überprüfen, kann, muss aber nicht durch die ATC übernommen werden.

Durch die Möglichkeiten einer ATC, mit Hilfe von signaltechnisch sicheren Ausgaben signaltechnisch sichere Funktionen zu realisieren, kann die Gefährdung für den Menschen durch fehlerhafte Aktivierung der Desinfektionssysteme signaltechnisch sicher überwacht und beherrscht werden. Durch die signaltechnische sichere Realisierung der Aktivierung/Deaktivierung durch eine ATC-Einrichtung kann diese Funktionalität bei höchstem Sicherungslevel (üblich im Bahnbetrieb) voll automatisiert werden (bis GoA4). Außerdem lässt sich eine Gefährdung von Menschen auf diesem Wege minimieren.

Die Aktivierungskriterien sind vorteilhaft weit skalierbar und können projektspezifisch definiert werden, so dass durch die ATC-Einrichtung am Ende ein signaltechnisch sicheres Kriterium ausgewertet werden kann. Die signaltechnisch sichere Ausgabe beispielsweise einer Freigabe für den Desinfektionsvorgang erhöht somit die Sicherheit, da bei der Übertragung der hierfür notwendigen Signale auftretende Fehler weitest möglich minimiert werden können.Durch die signaltechnisch sichere Aktivierung durch eine ATC kann außerdem vorteilhaft verhindert werden, dass ein Desinfektionsvorgang fehlerhaft während des Passagierbetriebs startet.

Zu definierten Zeitpunkten, wenn keine Passagiere an Bord sind (z.B. Züge außerhalb des Fahrgastbetriebs, Züge auf dem Weg ins Depot, automatische Kehrfahrt, Anforderung durch einen Operator) kann die fahrzeugseitige ATC-Einrichtung eine Desinfektion starten z.B. durch Aktivierung der Versorgungsspannung des Desinfektionssystems.

Das Desinfektionssystem kann seinerseits z.B. Statusdaten seiner Aktivität über das ATC-System protokollieren. Sollten die Bedingungen für eine Aktivierung des Desinfektionssystems nicht mehr gegeben sein (z.B., wenn man mit Menschen im Umfeld rechnen muss), dann kann die ATC-Einrichtung die Desinfektion signaltechnisch sicher deaktivieren.

Gemäß einer Ausgestaltung der Erfindung ist vorgesehen, dass der Desinfektionsvorgang durchgeführt wird, indem der Benutzerraum mit UV-Strahlung, insbesondere UV-C-Strahlung, bestrahlt wird.

Die Ultraviolettstrahlung (UV) wird in die Bereiche UV-A

(Wellenlänge 315-400 nm), UV-B (280-315 nm) und UV-C (100-280 nm) unterteilt. Das ultraviolette Licht (vorzugswies UV-C) greift die Viren an. Es dringt durch deren Hüllen, löst dort eine Reihe der wichtigsten chemischen Bindungen und unterbindet so die Vervielfältigung des Erbguts. Das Virus hat keine Möglichkeit mehr, sich zu vermehren und zu verbreiten. Es ist also nicht mehr virulent und faktisch abgeschaltet.

Die Desinfektion von Räumen mit z.B. UV-C Strahlung kann ein wirksames technisches Hilfsmittel der Zukunft für Innenbereiche des schienengebundenen Personenverkehrs, z.B. von Metro-Fahrzeugen, sein. Mit solchen Systemen können wirksam und schnell Viren auf Oberflächen (z.B. Haltestangen) und aus der Luft eliminiert werden. Der Desinfektionsvorgang ist allerdings gefährlich für den Menschen, deshalb muss dessen Aktivierung entsprechend überwacht werden, was das erfindungsgemäße Verfahren ermöglicht.

Gemäß einer Ausgestaltung der Erfindung ist vorgesehen, dass der Desinfektionsvorgang mit einer fest im Benutzerraum installierten Strahlungsquelle durchgeführt wird.

Eine fest installierte Strahlungsquelle hat den Vorteil, dass diese exakt auf die zu desinfizierenden Flächen ausgerichtet werden kann. Außerdem lässt sich dann eine Desinfektion jederzeit durchführen, wenn der Benutzerraum frei von Benutzern ist. Also auch beispielsweise in Wartezeiten eines leeren Schienenfahrzeugs. Es muss keine zusätzliche Maßnahme durchgeführt werden, um Mittel für die Desinfektionsmaßnahme in das Fahrzeug zu bringen.

Gemäß einer Ausgestaltung der Erfindung ist vorgesehen, dass die fest installierte Strahlungsquelle auf die Sitzflächen von Sitzen des Fahrzeugs und/oder Interface-Flächen für die Hände gerichtet wird.

Die Möglichkeit, die Strahlungsquelle auf bestimmte Flächen ausrichten zu können, macht die Desinfektionsmaßnahme vorteilhaft besonders wirkungsvoll. Beispielsweise die Sitzflächen aber auch andere Interface-Flächen wie Haltegriffe oder Haltestangen aber auch Türklinken oder Bedienelemente werden durch die Benutzer am ehesten kontaminiert, sodass die Hebelwirkung einer Desinfektion an diesen Stellen besonders groß ist.

Gemäß einer Ausgestaltung der Erfindung ist vorgesehen, dass der Benutzerraum mit einer Überwachungsvorrichtung auf die Abwesenheit von Benutzern hin überwacht wird.

Die Überwachungsvorrichtung kann einerseits vorteilhaft dazu genutzt werden, die Benutzerfreiheit des Benutzerraums festzustellen, bevor der Desinfektionsvorgang gestartet wird. Vorteilhaft kann die Überwachungsvorrichtung allerdings auch sicherstellen, dass während des Desinfektionsvorgangs kein Benutzer in den Benutzerraum eindringt und sich somit selbst gefährdet.

Die Überwachungsvorrichtung kann mit jeder Art von Sensoren ausgestattet sein, welche es ermöglichen, Benutzer im Benutzerraums festzustellen. Außerdem muss die Überwachungsvorrichtung mit einer geeigneten Auswertungseinheit verbunden sein, damit die Sensorergebnisse entsprechend interpretiert werden können. Diese Auswertungseinheit kann in der Infrastruktur vorgesehen sein, die das Fahrzeug, der Roboter oder beispielsweise ein Bahnhof zur Verfügung stellt. Andererseits kann die Auswertungseinheit auch durch einen hierfür zur Verfügung stehenden Computer gebildet sein.

Gemäß einer Ausgestaltung der Erfindung ist vorgesehen, dass als Überwachungsvorrichtung eine Kamera genutzt wird, mit der der Benutzerraum im Wellenlängenbereich des sichtbaren Lichtes und/oder von Infrarotlicht abgebildet wird.

Als Überwachungsvorrichtung eignen sich Kameras in besonderer Weise, da durch diese eine Anwesenheit von Benutzern gut festgestellt werden kann. Hierbei können entweder Kameras verwendet werden, welche ein sichtbares Bild aufzeichnen und/oder Infrarotkameras. Die Kamerabilder können durch Personal ausgewertet werden oder es erfolgt eine automatisierte Bildauswertung.

Besonderes gilt für Kameras im Infrarotlichtbereich. Auch hier können die Bilder ausgewertet werden. Infrarotbilder haben hierbei den Vorteil, dass sich die Anwesenheit von Benutzern aufgrund ihrer Körpertemperatur sehr gut feststellen lässt und es auch möglich ist, dass diese erkannt werden, wenn sie sich beispielsweise hinter einem Vorhang befinden.

Gemäß einer Ausgestaltung der Erfindung ist vorgesehen, dass sich während des Desinfektionsvorganges die Türen nicht oder nur von innen öffnen lassen.

Diese Maßnahme schafft vorteilhaft eine zusätzliche Sicherheit, da der Benutzerraum, der durch den Innenraum des Fahrzeugs gebildet wird, während des Desinfektionsvorgangs dann nicht betreten werden kann. Eine versehentliche Kontaminierung von Benutzern kann damit ausgeschlossen werden und die Gefahr, dass ein Desinfektionsvorgang aus Sicherheitsgründen abgebrochen werden muss kann wesentlich verringert werden.

Die genannte Aufgabe wird alternativ mit dem eingangs angegebenen Anspruchsgegenstand (Fahrzeug) erfindungsgemäß auch dadurch gelöst, dass der Desinfektionsvorgang automatisch durchführbar ist und vor dem automatischen Durchführen der Desinfektionsmaßnahmen rechnergestützt eine Beurteilung durchführbar ist, ob der Benutzerraum frei von Benutzern ist, wobei die Desinfektionsvorgang nur gestartet wird, wenn die Beurteilung zu dem Ergebnis geführt hat, dass der Benutzerraum frei von Benutzern ist.

Insbesondere kann in dem erfindungsgemäßen Fahrzeug eine fest installierte Einrichtung zur Desinfektion, insbesondere eine Strahlungsquelle, zum Einsatz kommen. Alternativ ist selbst verständlich auch der Einsatz eines Roboters (mobile Einheit) denkbar.

Die genannte Aufgabe wird alternativ mit dem eingangs angegebenen Anspruchsgegenstand (mobile Einheit) erfindungsgemäß auch dadurch gelöst, dass der Desinfektionsvorgang automatisch durchführbar ist und vor dem automatischen Durchführen der Desinfektionsmaßnahmen rechnergestützt eine Beurteilung durchführbar ist, ob der Benutzerraum frei von Benutzern ist, wobei die Desinfektionsvorgang nur gestartet wird, wenn die Beurteilung zu dem Ergebnis geführt hat, dass der Benutzerraum frei von Benutzern ist.

Mit den Vorrichtungen (Fahrzeug, mobile Einheit) lassen sich die Vorteile erreichen, die im Zusammenhang mit dem obenstehend näher beschriebenen Verfahren bereits erläutert wurden. Das zum erfindungsgemäßen Verfahren Aufgeführte gilt entsprechend auch für die erfindungsgemäßen Vorrichtungen.

Des Weiteren wird ein Computerprogrammprodukt mit Programmbefehlen zur Durchführung des genannten erfindungsgemäßen Verfahrens und/oder dessen Ausführungsbeispielen beansprucht, wobei mittels des Computerprogrammprodukts jeweils das erfindungsgemäße Verfahren und/oder dessen Ausführungsbeispiele durchführbar sind.

Darüber hinaus wird eine Bereitstellungsvorrichtung zum Speichern und/oder Bereitstellen des Computerprogrammprodukts beansprucht. Die Bereitstellungsvorrichtung ist beispielsweise ein Speichereinheit, die das Computerprogrammprodukt speichert und/oder bereitstellt. Alternativ und/oder zusätzlich ist die Bereitstellungsvorrichtung beispielsweise ein Netzwerkdienst, ein Computersystem, ein Serversystem, insbesondere ein verteiltes, beispielsweise cloudbasiertes Computersystem und/oder virtuelles Rechnersystem, welches das Computerprogrammprodukt vorzugsweise in Form eines Datenstroms speichert und/oder bereitstellt.

Die Bereitstellung erfolgt in Form eines Programmdatenblocks als Datei, insbesondere als Downloaddatei, oder als Datenstrom, insbesondere als Downloaddatenstrom, des Computerprogrammprodukts. Diese Bereitstellung kann beispielsweise aber auch als partieller Download erfolgen, der aus mehreren Teilen besteht. Ein solches Computerprogrammprodukt wird beispielsweise unter Verwendung der Bereitstellungsvorrichtung in ein System eingelesen, sodass das erfindungsgemäße Verfahren auf einem Computer zur Ausführung gebracht wird.

Weitere Einzelheiten der Erfindung werden nachfolgend anhand der Zeichnung beschrieben. Gleiche oder sich entsprechende Zeichnungselemente sind jeweils mit den gleichen Bezugszeichen versehen und werden nur insoweit mehrfach erläutert, wie sich Unterschiede zwischen den einzelnen Figuren ergeben.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Komponenten auch durch mit den vorstehend beschriebenen Merkmalen der Erfindung kombinierbar.

Es zeigen:
Figur 1 ein Ausführungsbeispiel der erfindungsbemäßen Vorrichtungen (Fahrzeug und Roboter) mit ihren Wirkzusammenhängen schematisch,
Figur 2 ein Ausführungsbeispiel der Computer-Infrastruktur der Vorrichtung gemäß Figur 1 als Blockschaltbild, wobei die einzelnen Funktionseinheiten als Programmmodule jeweils in einem oder mehreren Prozessoren ablaufen können und die Schnittstellen demgemäß softwaretechnisch oder hardwaretechnisch ausgeführt sein können,
Figur 3 ein Ausführungsbeispiel des erfindungsgemäßen Verfahrens als Flussdiagramm, wobei die Funktionseinheiten und Schnittstellen gemäß Figur 2 beispielhaft angedeutet sind.

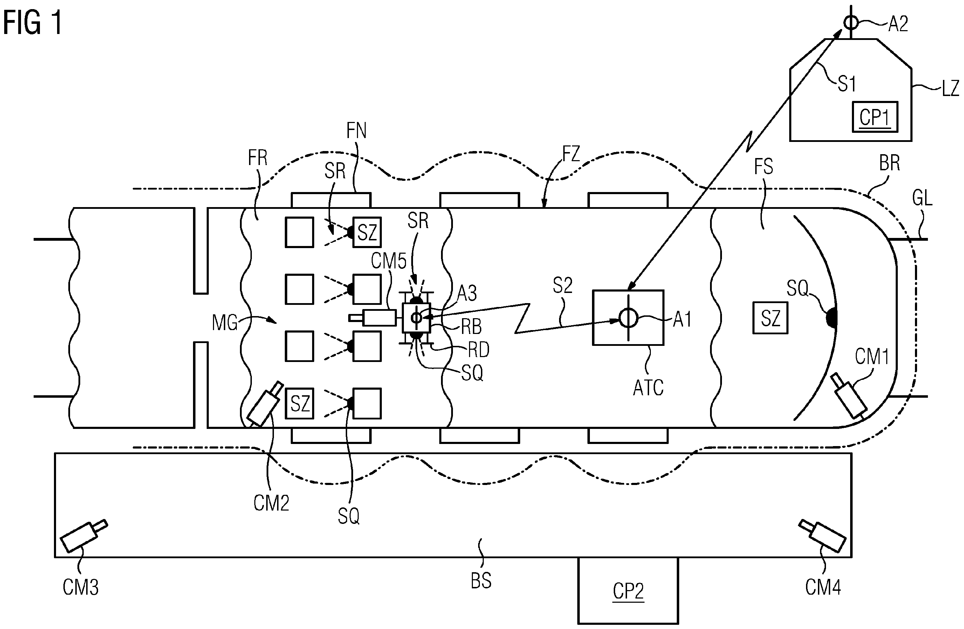

Gemäß Figur 1 ist ein Fahrzeug FZ dargestellt, welches auf einem Gleis GL steht. Außerdem steht das Fahrzeug FZ an einem Bahnsteig BS in einer Parkposition.

Das Fahrzeug FZ ist von oben und teilweise aufgebrochen dargestellt, sodass man sowohl in einen Fahrstand FS als auch in einen Fahrgastraum FR hineinsehen kann. In dem Fahrstand FS und dem Fahrgastraum FR sind Sitze SZ dargestellt, die zumindest im Fahrgastraum FR auch einen Mittelgang MG bilden. Außerdem sind Fenster FN angedeutet, die sich in der Wand des Fahrzeugs FZ befinden.

Erfindungsgemäß ist das Fahrzeug FZ dazu ausgelegt, dass in diesem ein Desinfektionsvorgang durchgeführt werden kann. Zu diesem Zweck sind Strahlungsquellen SQ vorgesehen, welche eine Strahlung SR in Form von UV-B-Strahlung erzeugen können. Beispielsweise sind Strahlungsquellen SQ jeweils auf die Sitze SZ gerichtet, um die Sitzflächen, mit denen Fahrgäste oder ein Zugführer direkt in Kontakt stehen, zu desinfizieren. Dafür können im Fahrgastraum FR z. B. die Strahlungsquellen SQ an den Rückenlehnen der vor den zu desinfizierenden Sitzen gelegenen Sitze angebracht sein.

Eine andere Möglichkeit besteht darin, einen Roboter RB durch den Mittelgang MG zu führen. Dieser ist mit Rädern RD ausgestattet und besitzt ebenfalls Strahlungsquellen SQ. Außerdem weist der Roboter RB eine Kamera CM5 auf, die eine autonome Steuerung der Bewegung des Roboters RB unterstützt. Allerdings ist der Roboter RB auch über eine zweite Schnittstelle S2 mit einer automatischen Zugsteuerung ATC verbunden. Hierbei kommen eine erste Antenne A1 und eine dritte Antenne A3 zum Einsatz.

Die automatische Zugsteuerung ATC kann auch mit einer Leitzentrale LZ über eine zweite Antenne A2 eine Verbindung aufbauen, die durch eine erste Schnittstelle S1 angedeutet ist. Bei der ersten Schnittstelle S1 und der zweiten Schnittstelle S2 handelt es sich somit um Funkschnittstellen.

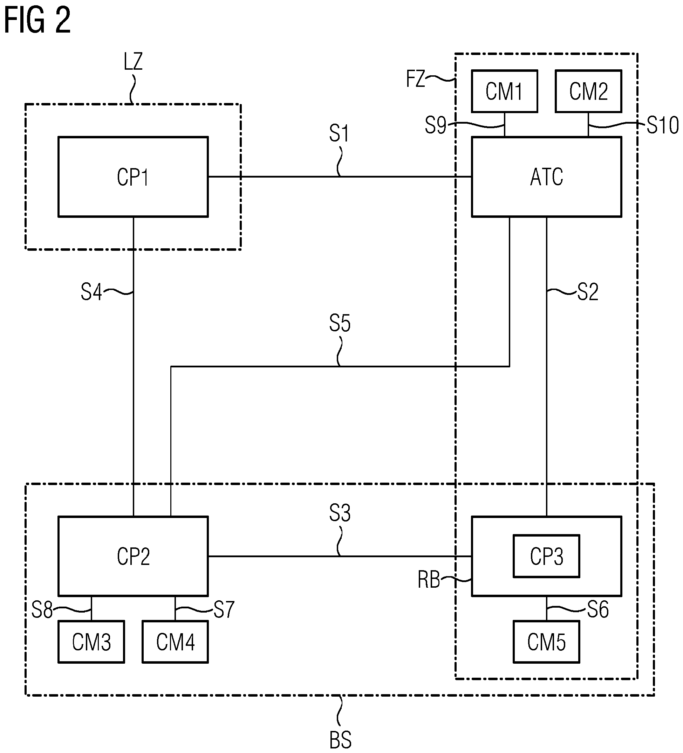

Um das erfindungsgemäße Verfahren durchzuführen, kommen verschiedene Computer zum Einsatz (vgl. auch Figur 2). In der Leitzentrale LZ ist ein erster Computer CP1 vorgesehen. Auch der Bahnsteig BS ist mit einem zweiten Computer CP2 ausgestattet. Auch die automatische Zugsteuerung ATC ist durch einen Computer ausgebildet. In dem Roboter RB kommt ein in Figur 1 nicht dargestellter dritter Computer CP3 zum Einsatz.

Die genannten Computer steuern unter anderem eine dritte Kamera CM3 und eine vierte Kamera CM4 auf dem Bahnsteig BS, eine erste Kamera CM1 in dem Fahrstand FS und eine zweite Kamera CM2 im Fahrgastraum FR an. Die hierzu zum Einsatz kommenden Schnittstellen S6 ... S10 sind in Figur 2 dargestellt, woraus erkenntlich wird, welche Kameras welchem Computer zugeordnet sind. Die Kameras dienen der Überwachung des Desinfektionsvorgangs, der im Folgenden näher erläutert wird.

Während des Desinfektionsvorgangs durch die Strahlung SR muss ein Benutzerraum BR frei von Benutzern gehalten werden. Um dies zu überwachen, werden die Kameras CM1 ... CM5 verwendet, wobei die fünfte Kamera CM5 gleichzeitig verwendet wird, damit der Roboter RB eventuell in seinem Weg vorhandene Hindernisse erkennen kann. Der Benutzerraum BR wird gebildet durch den Innenraum des Fahrzeugs FZ, welcher sowohl den Fahrstand FS als auch den Fahrgastraum FR umfasst. Außerdem kann durch die Fenster FN Strahlung SR aus dem Inneren des Fahrzeugs FZ nach außen dringen, weshalb beispielsweise an der Kante des Bahnsteigs BS befindliche Personen Schaden nehmen könnten. Deswegen ist der Benutzerraum BR als eine Umhüllende des Fahrzeugs FZ dargestellt, wobei um die Fenster FN herum in der Umgebung des Fahrzeugs FZ ein Raum definiert ist, in dem eventuell aus den Fenstern FN dringende Strahlung SR schädlich für Benutzer sein könnte. Dies macht deutlich, dass der Benutzerraum BR auch außerhalb des Fahrzeugs liegen kann und dessen Definition nicht unbedingt von der Benutzung des Fahrzeugs im bestimmungsgemäßen Betrieb definiert ist, sondern von der Gefährdungslage, die sich während des Desinfektionsvorgangs ergibt.

Der Figur 2 lässt sich entnehmen, wie die Computer CP1 ... CP3 sowie die automatische Zugsteuerung ATC miteinander kommunizieren können. Der erste Computer CP1 der Leitzentrale LZ ist, wie bereits in Figur 1 dargestellt, über die erste Schnittstelle S1 mit der automatischen Zugsteuerung ATC verbunden. Ebenso ist die zweite Schnittstelle S2 zwischen der automatischen Zugsteuerung ATC und dem dritten Computer CP3 des Roboters RB bereits in Figur 1 angesprochen worden. Mittels dieser Schnittstelle ist es beispielsweise möglich, dass der Roboter RB zumindest teilweise von der automatischen Zugsteuerung ATC gesteuert wird. Es ist auch möglich, dem Roboter RB lediglich Daten zu dessen Selbststeuerung über die zweite Schnittstelle S2 zu übermitteln.

Auch mit dem zweiten Computer CP2 des Bahnsteigs BS kann der dritte Computer CP3 des Roboters RB über eine dritte Schnittstelle S3 kommunizieren. Hierdurch ist es beispielsweise möglich, dass der Roboter RB Daten der dritten Kamera CM3 und der vierten Kamera CM4 auswertet und beispielsweise den Desinfektionsvorgang stoppt, wenn außerhalb des Zuges in den Benutzerraum BR auf dem Bahnsteig BS Personen eindringen.

Der zweite Computer CP2 des Bahnsteigs BS kann über eine vierte Schnittstelle S4 mit dem ersten Computer CP1 der Leitzentrale LZ sowie über eine fünfte Schnittstelle S5 mit der automatischen Zugsteuerung ATC des Fahrzeugs FZ kommunizieren.

Es wird somit deutlich, dass ein Netzwerk von Computern zur Verfügung steht, die insbesondere aus einer bestehenden Infrastruktur, nämlich aus der Leitzentrale LZ, dem Bahnhof (d. h. Bahnsteig BS), dem Roboter RB und dem Fahrzeug selbst (bzw. dessen automatische Zugsteuerung ATC) besteht. Diese Computerinfrastruktur kann genutzt werden, um den Desinfektionsvorgang zu steuern und eine Gefährdung von Benutzern zu überwachen. Dabei können die Aufgaben des rechnergestützten Verfahrens auf die dargestellten Computer oder einen Teil der dargestellten Computer oder zusätzlich auf weitere, nicht dargestellte, Computer verteilt werden.

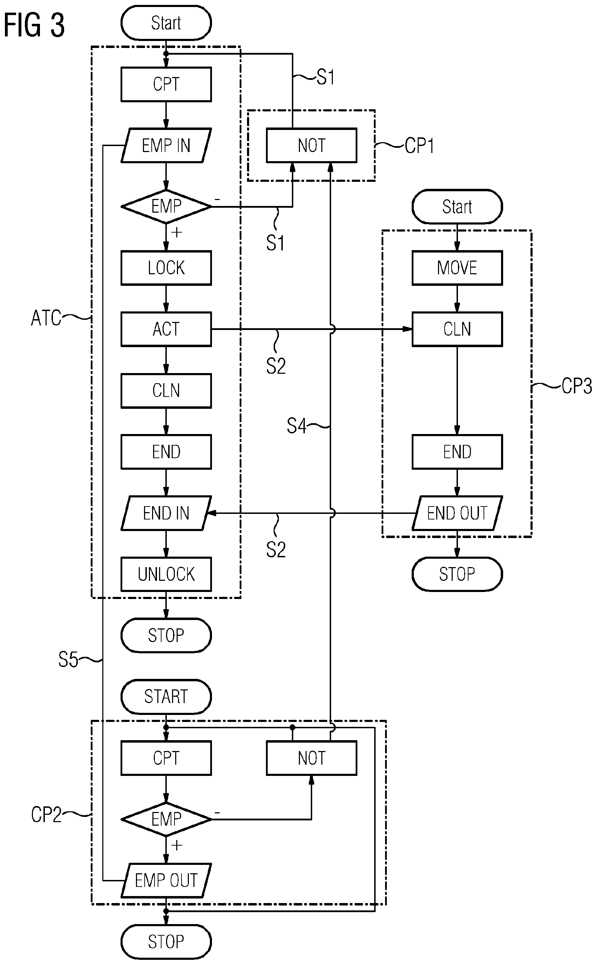

Der Figur 3 kann das Verfahren zum Reinigen des Zuges, welches auch den Desinfektionsvorgang CLN enthält, entnommen werden. Das Verfahren in der automatischen Zugsteuerung ATC und im zweiten Computer CP2 des Bahnsteigs BS startet mit START gleichzeitig. Der Computer CP1 der Leitzentrale LZ steht mit seiner Rechenkapazität währenddessen immer zur Verfügung.

Sowohl durch die Zugsteuerung ATC als auch den zweiten Computer CP2 werden parallel Erfassungsschritte CPT durchgeführt, wobei beispielsweise mit den in Figur 1 dargestellten Kameras CM1 ... CM4 erfasst werden kann, ob der Benutzerraum BR frei von Benutzern ist. Anschließend erfolgt im Computer CP2 ein Abfrageschritt EMP, ob der Benutzerraum BR leer ist. Im negativen Fall erfolgt ein Notifikationsschritt NOT, dass der Computer CP2 den Bahnsteig weiter überwachen muss, und der Erfassungsschritt CPT wird wiederholt. Erst, wenn der Abfrageschritt EMP ein positives Ergebnis (d. h. Benutzerraum leer) ergibt, erfolgt ein Ausgabeschritt EMP OUT, dass der Benutzerraum BR auf dem Bahnsteig BS leer ist, und wird über die fünfte Schnittstelle S5 an die automatische Zugsteuerung ATC gegeben (dort erfolgt ein Eingabeschritt EMP IN, dass der Benutzerraum leer ist).

Allerdings erfolgt auch in der automatischen Zugsteuerung ATC eine Auswertung des Erfassungsschritts für das Fahrzeug CPT in der beschriebenen Weise. Wenn der Abfrageschritt EMP negativ ausfällt, erfolgt über die erste Schnittstelle S1 ein Notifikationsschritt NOT, der in dem ersten Computer CP1 der Leitzentrale LZ stattfindet. Diese sendet dann eine Anforderung über die erste Schnittstelle S1, dass der Erfassungsschritt CPT wiederholt werden muss.

Erzeugt der Abfrageschritt EMP ein positives Ergebnis, so wird anschließend ein Verriegelungsschritt für die Türen LOCK durchgeführt. Damit kann sichergestellt werden, dass der Benutzerraum BR im Inneren des Fahrzeugs FZ nicht durch Benutzer betreten werden kann. Anschließend wird in einem Aktivierungsschritt ACT der Roboter RB aktiviert, der vorher schon gestartet wurde und in einem Bewegungsschritt MOVE an seine Startposition für den Desinfektionsvorgang CLN gerollt wurde. Nach dem Aktivierungsschritt ACT kann dieser den Desinfektionsvorgang CLN vornehmen. Während des Desinfektionsvorgangs CLN, der durch den Roboter RB durchgeführt wird, steuert die automatische Zugsteuerung ATC gleichzeitig die fest im Fahrzeug FZ installierten Strahlungsquellen SQ (vgl. Figur 1) an, die ebenfalls an dem Desinfektionsvorgang CLN beteiligt sind.

Ist der Desinfektionsvorgang CLN beendet, so erfolgt sowohl in der automatischen Zugsteuerung ATC als auch in dem Computer CP3 des Roboters RB ein Quittierungsschritt END. Nach Quittierung erfolgt ein Ausgabeschritt der Quittierung END OUT über die zweite Schnittstelle S2 an die automatische Zugsteuerung ATC, wo dies einen Eingabeschritt END IN auslöst. Die automatische Zugsteuerung ATC wartet somit sowohl auf den ATC-internen Quittierungsschritt END und den Eingabeschritt END IN. Wenn beide vorliegen, erfolgt ein Entriegelungsschritt UNLOCK für die Türen. Die Prozesse können nun gestoppt werden (STOP).

Während des Desinfektionsvorgangs CLN läuft nach dem Ausgabeschritt EMP OUT im Computer CP2 eine Rekursion ab, damit der Erfassungsschritt CPT zur Überwachung des Bahnsteigs BS fortgeführt wird. Für den Fall, dass aufgrund eines Eindringens eines Benutzers in den Benutzerraum BR auf dem Bahnsteig BS eine Gefährdung entsteht, kann die bereits angesprochene Notifikation (Notifikationsschritt NOT) über die vierte Schnittstelle S4 an die Leitzentrale LZ bzw. den dort zum Einsatz kommenden Computer CP1 gegeben werden. Dieser erzeugt einen Notifikationsschritt NOT, der an die automatische Zugsteuerung ATC gegeben werden kann und dort aus Sicherheitsgründen einen in Figur 3 nicht näher dargestellten Abbruch des Desinfektionsvorgangs CLN auslöst.

Figur 3 zeigt ein Beispiel, bei dem sowohl eine fest installierte Desinfektionsvorrichtung als auch der Roboter RB zum Einsatz kommt. Denkbar sind auch Verfahren, bei denen nur eine fest installierte Vorrichtung im Fahrzeug zum Einsatz kommt oder nur ein Roboter RB, wobei die Darstellung in Figur 3 lediglich dahingehend modifiziert werden müsste, dass der entsprechende Desinfektionsvorgang CLN sowie evtl. die Kommunikation über die zweite Schnittstelle S2 weggelassen werden würde.

### Bezugszeichenliste

- LZ: Leitzentrale
- GL: Gleis
- FZ: Fahrzeug
- FN: Fenster
- SZ: Sitz
- MG: Mittelgang
- CM1 ... CM5: Kamera

- FR: Fahrgastraum
- FS: Fahrstand
- BS: Bahnsteig
- BR: Benutzerraum

- RB: Roboter
- RD: Rad
- SQ: Strahlungsquelle
- SR: Strahlung
- ATC: Automatische Zugsteuerung
- CP1 ... CP3: Computer
- A1 ... A3: Antenne
- S1 ... S10: Schnittstelle

- CPT: Erfassungsschritt
- NOT: Notifikationsschritt
- EMP IN: Eingabeschritt (Bahnsteig leer)
- EMP OUT: Ausgabeschritt (Bahnsteig leer)
- EMP: Abfrageschritt (Benutzerraum leer)
- LOCK: Verriegelungsschritt für Türen
- ACT: Aktivierungsschritt (Roboter)
- UNLOCK: Entriegelungsschritt für Türen
- MOVE: Bewegungsschritt für Roboter
- END: Quittierungsschritt
- END OUT: Ausgabeschritt (Quittierung)
- END IN: Eingabeschritt (Quittierung)
- CLN: Desinfektionsvorgang

## Patentansprüche

1. Verfahren zum Desinfizieren eines Benutzerraumes (BR) in einem Fahrzeug (FZ), bei dem in dem Benutzerraum (BR) ein Desinfektionsvorgang (CLN) durchgeführt wird, wobei
• der Desinfektionsvorgang (CLN) automatisch durchgeführt wird,
• vor dem automatischen Durchführen des Desinfektionsvorgangs (CLN) rechnergestützt eine Beurteilung durchgeführt wird, ob der Benutzerraum (BR) frei von Benutzern ist, wobei die Desinfektionsvorgang (CLN) nur gestartet wird, wenn die Beurteilung zu dem Ergebnis geführt hat, dass der Benutzerraum (BR) frei von Benutzern ist
• der Desinfektionsvorgang (CLN) durchgeführt wird, indem durch den Benutzerraum (BR) eine als Roboter (RB) ausgeführte mobile Einheit durch ein in den Roboter (RB) integriertes Steuersystem geführt wird,
**dadurch gekennzeichnet,**
**dass** der Roboter (RB) über eine Schnittstelle (S1 ... S10) mit der automatischen Zugsteuerung (ATC) des Fahrzeugs (FZ) kommuniziert.

2. Verfahren nach Anspruch 1
**dadurch gekennzeichnet,**
**dass** die Aktivierung eines Desinfektionsvorgangs (CLN) durch eine fahrzeugseitige automatische Zugsteuerung (ATC) oder automatische Zugsicherung eines Fahrzeugs (FZ) mittels eines signaltechnisch sicheren Ausgangs vorgenommen wird, nachdem diese ein Signal erhalten hat, welches darauf hinweist, dass der Benutzerraum (BR) frei von Benutzern ist.

3. Verfahren nach einem der voranstehenden Ansprüche,
**dadurch gekennzeichnet,**
**dass** der Desinfektionsvorgang (CLN) durchgeführt wird, indem der Benutzerraum (BR) mit UV-Strahlung (SR), insbesondere UV-C-Strahlung, bestrahlt wird.

4. Verfahren nach Anspruch 3,
**dadurch gekennzeichnet,**
**dass** der Desinfektionsvorgang (CLN) mit einer fest im Benutzerraum (BR) installierten Strahlungsquelle (SQ) durchgeführt wird.

5. Verfahren nach Anspruch 4,
**dadurch gekennzeichnet,**
**dass** die fest installierte Strahlungsquelle (SQ) auf die Sitzflächen von Sitzen (SZ) des Fahrzeugs (FZ) und/oder Interface-Flächen für die Hände gerichtet wird.

6. Verfahren nach einem der voranstehenden Ansprüche,
**dadurch gekennzeichnet,**
**dass** der Benutzerraum (BR) mit einer Überwachungsvorrichtung auf die Abwesenheit von Benutzern hin überwacht wird.

7. Verfahren nach Anspruch 6,
**dadurch gekennzeichnet,**
**dass** als Überwachungsvorrichtung eine Kamera (CM1 ... CM5) genutzt wird, mit der der Benutzerraum (BR) im Wellenlängenbereich des sichtbaren Lichtes und/oder von Infrarotlicht abgebildet wird.

8. Verfahren nach einem der voranstehenden Ansprüche,
**dadurch gekennzeichnet,**
**dass** sich während des Desinfektionsvorganges (CLN) die Türen nicht oder nur von innen öffnen lassen.

9. Verfahren nach einem der voranstehenden Ansprüche,
**dadurch gekennzeichnet,**
**dass** die rechnergestützte Beurteilung, ob der Benutzerraum (BR) frei von Benutzern ist, derart durchgeführt wird, dass auf die Eingabe eines die Benutzerfreiheit des Benutzerraumes (BR) anzeigenden Signals gewartet wird.

10. Fahrzeug (FZ) mit einem Roboter gemäß Anspruch 11 , das eingerichtet ist, ein Verfahren zum Desinfizieren eines Benutzerraumes (BR) des Fahrzeugs (FZ) gemäß einem der Ansprüche 1 bis 9 durchzuführen.

11. Roboter, der eingerichtet ist, ein Verfahren zum Desinfizieren eines Benutzerraumes (BR) eines Fahrzeugs (FZ) gemäß einem der Ansprüche 1 bis 9 durchzuführen.

12. Computerprogrammprodukt mit Programmbefehlen zur Durchführung des Verfahrens nach einem der Ansprüche 1 - 9.

13. Bereitstellungsvorrichtung für das Computerprogrammprodukt nach dem letzten voranstehenden Anspruch, wobei die Bereitstellungsvorrichtung das Computerprogrammprodukt speichert und/oder bereitstellt.

## Claims

1. Method for disinfecting a user space (BR) in a vehicle (FZ), in which a disinfection process (CLN) is carried out in the user space (BR), wherein
• the disinfection process (CLN) is carried out automatically,
• prior to the disinfection process (CLN) being carried out automatically, an assessment is made in a computer-assisted manner as to whether the user space (BR) is unoccupied by users, wherein the disinfection process (CLN) is only started if the assessment produces the result that the user space (BR) is unoccupied by users
• the disinfection process (CLN) is carried out by a mobile unit configured as a robot (RB) being guided through the user space (BR) by way of a control system integrated in the robot (RB),
**characterised in that**
the robot (RB) communicates with the automatic train control system (ATC) of the vehicle (FZ) via an interface (S1 ... S10).

2. Method according to claim 1,
**characterised in that**
the activation of a disinfection process (CLN) is carried out by an automatic train control system (ATC) on the vehicle or an automatic train protection system of a vehicle (FZ) by means of an output which is failsafe in terms of signalling, after it receives a signal which indicates that the user space (BR) is unoccupied by users.

3. Method according to one of the preceding claims,
**characterised in that**
the disinfection process (CLN) is carried out by the user space (BR) being irradiated with UV radiation (SR), in particular UV-C radiation.

4. Method according to claim 3,
**characterised in that**
the disinfection process (CLN) is carried out with a radiation source (SQ) which is permanently installed in the user space (BR).

5. Method according to claim 4,
**characterised in that**
the permanently installed radiation source (SQ) is directed onto the seat surfaces of seats (SZ) of the vehicle (FZ) and/or the interface surfaces for hands.

6. Method according to one of the preceding claims,
**characterised in that**
the user space (BR) is monitored for the absence of users with a monitoring apparatus.

7. Method according to claim 6,
**characterised in that**
a camera (CM1 ... CM5) is used as the monitoring apparatus, with which the user space (BR) is imaged in the wavelength region of visible light and/or with infrared light.

8. Method according to one of the preceding claims,
**characterised in that**
the doors cannot be opened or may only be opened from the inside during the disinfection process (CLN).

9. Method according to one of the preceding claims,
**characterised in that**
the computer-assisted assessment as to whether the user space (BR) is unoccupied by users is carried out pending the input of a signal which indicates that the user space (BR) is unoccupied by users.

10. Vehicle (FZ) with a robot according to claim 11, which is designed to carry out a method for disinfecting a user space (BR) of the vehicle (FZ) according to one of claims 1 to 9.

11. Robot which is designed to carry out a method for disinfecting a user space (BR) of a vehicle (FZ) according to one of claims 1 to 9.

12. Computer program product with program commands for carrying out the method according to one of claims 1 - 9.

13. Provision apparatus for the computer program product according to the last preceding claim, wherein the provision apparatus stores and/or provides the computer program product.

## Revendications

1. Procédé de désinfection d'un espace (BR) des utilisateurs dans un véhicule (FZ), dans lequel dans l'espace (BR) pour des utilisateurs on effectue une opération (CLN) de désinfection, dans lequel
• on effectue l'opération (CLN) de désinfection automatiquement,
• avant d'effectuer automatiquement l'opération (CLN) de désinfection, on donne en se faisant assister par un ordinateur un jugement sur le point de savoir si l'espace (BR) des utilisateurs est inoccupé par des utilisateurs, dans lequel on ne lance l'opération (CLN) de désinfection que si le jugement a donné le résultat que l'espace (BR) des utilisateurs est inoccupé par des utilisateurs
• on effectue l'opération (CLN) de désinfection en guidant, par un système de commande intégrée dans le robot (RB), dans l'espace (BR) des utilisateurs une unité mobile réalisée sous la forme d'un robot (RB),
**caractérisé,**
**en ce que** le robot (RB) communique par une interface (S1 ... S10) avec le pilotage automatique des trains (ATC) ou avec la protection automatique des trains du véhicule (FZ).

2. Procédé suivant la revendication 1
**caractérisé,**
**en ce qu'**on effectue l'activation d'une opération (CLN) de désinfection par un pilotage automatique des trains (ATC) embarqué sur le véhicule ou par une protection automatique des trains d'un véhicule (FZ) au moyen d'une sortie sécurisée en technique du signal après que celle-ci a reçu un signal, qui indique que l'espace (BR) des utilisateurs est inoccupé par des utilisateurs.

3. Procédé suivant l'une des revendications précédentes,
**caractérisé,**
**en ce qu'**on effectue l'opération (CLN) de désinfection en exposant l'espace (BR) des utilisateurs à du rayonnement UV (SR), en particulier à du rayonnement UV-C.

4. Procédé suivant la revendication 3,
**caractérisé,**
**en ce qu'**on effectue l'opération (CLN) de désinfection par une source (SQ) de rayonnement montée fixe dans l'espace (BR) des utilisateurs.

5. Procédé suivant la revendication 4,
**caractérisé,**
**en ce que** l'on dirige la source (SQ) de rayonnement montée fixe sur les surfaces d'assise de sièges (SZ) du véhicule (FZ) et/ou sur des surfaces d'interface pour les mains.

6. Procédé suivant l'une des revendications précédentes,
**caractérisé,**
**en ce que** l'on contrôle l'absence d'utilisateur dans l'espace (BR) des utilisateurs par un dispositif de contrôle.

7. Procédé suivant la revendication 6,
**caractérisé,**
**en ce que** l'on utilise comme dispositif de contrôle une caméra (CM1 ... CM5), par laquelle on représente l'espace (BR) des utilisateurs dans le domaine de longueur d'onde de la lumière visible et/ou de la lumière infrarouge.

8. Procédé suivant l'une des revendications précédentes,
**caractérisé,**
**en ce que** pendant l'opération (CLN) de désinfection, on ne laisse pas les portes ouvertes ou on ne les laisse ouvertes que de l'intérieur.

9. Procédé suivant l'une des revendications précédentes,
**caractérisé,**
**en ce que** l'on effectue le jugement assisté par ordinateur du point de savoir si l'espace (BR) des utilisateurs n'est pas occupé par des utilisateurs de telle manière que l'on attend l'entrée d'un signal indiquant l'inoccupation par les utilisateurs de l'espace (BR) des utilisateurs.

10. véhicule (FZ) comprend un robot suivant la revendication 11, qui est agencé pour effectuer un procédé de désinfection d'un espace (BR) des utilisateurs du véhicule (FZ), suivant l'une des revendications 1 à 9.

11. Robot, qui est agencé pour effectuer un procédé de désinfection d'un espace (BR) des utilisateurs d'un véhicule (FZ) suivant l'une des revendications 1 à 9.

12. Produit de programme d'ordinateur comprenant des instructions de programme pour effectuer le procédé suivant l'une des revendications 1 à 9.

13. Dispositif de mise à disposition du produit de programme d'ordinateur suivant la dernière revendication précédente, le dispositif de mise à disposition mettant en mémoire et/ou mettant à disposition le produit de programme d'ordinateur.
